# EUROPÄISCHE PATENTSCHRIFT

(11) **EP 2 965 701 B1**
(45) Veröffentlichungstag und Bekanntmachung des Hinweises auf die Patenterteilung: **27.02.2019**
(21) Anmeldenummer: 14176815.0
(22) Anmeldetag: 11.07.2014
(51) Int. Cl.: A61B 17/70

(54) **Stabfassklemme**
Rod-type barrel clamp
Pince à tige

(43) Veröffentlichungstag der Anmeldung: 13.01.2016
(73) Patentinhaber: Silony Medical International AG, 8500 Frauenfeld (CH)
(72) Erfinder: Trautwein, Frank Thilo, 70794 Filderstadt (DE); Heuer, Frank, 70794 Filderstadt (DE); Faas, Kai, 71364 Winnenden (DE)
(74) Vertreter: Stolmár & Partner Patentanwälte PartG mbB

(56) Entgegenhaltungen:
- DE-A1- 1 810 806
- DE-T2- 60 130 512
- FR-A1- 2 657 246
- US-A1- 2013 150 905
- US-A1- 2014 107 706

## Beschreibung

### Gebiet der Erfindung

Die Erfindung betrifft eine Stabfassklemme zum Halten und Bewegen eines Querstabs, mit dem Verankerungselemente wie bspw. Pedikelschrauben miteinander verbunden werden.

### Stand der Technik

Herkömmlicherweise werden zur Positionierung von Verankerungselementen wie mono- oder polyaxialen Pedikelschrauben Stabfasszangen verwendet. Dazu werden die Pedikelschrauben an den vorgesehenen Stellen in dem Wirbel der Wirbelsäule verankert und über einen Stab miteinander verbunden. Der Stab ist dabei je nach Anforderung schon vorgebogen oder wird durch den Anwender konturiert, da die Verankerungselemente nicht nur zur Stabilisierung von zwei Wirbeln dienen, sondern auch um Schiefstellungen des Rückgrats zu korrigieren. Dazu wird der Querstab in die jeweiligen Verankerungselemente eingesetzt, wobei seine Krümmung in etwa der Krümmung der Wirbelsäule entspricht, um das Einsetzen des Stabs einfach zu gestalten. Dann wird der Stab mit einer Stabfasszange 100 umfasst und durch Verschieben und Verdrehen des Stabes die beiden Verankerungselemente in die gewünschte Position und Orientierung gebracht. Das bedeutet, dass durch das Verschieben und Verdrehen des Querstabs die Verankerungselemente und damit auch die mit den Verankerungselementen verbundenen Wirbel in eine gewünschte Position gebracht werden. Dann werden die jeweiligen Fixierschrauben in den Verankerungselementen festgedreht, so dass der Querstab in seiner gewünschten Position mit den Verankerungselementen stabil befestigt ist. Am Ende können dann noch die Enden des Querstabs, die aus den jeweiligen Verankerungselementen überstehen, mit einer speziellen Zange oder einer Fräse abgezwickt, abgeschnitten oder anders entfernt werden.

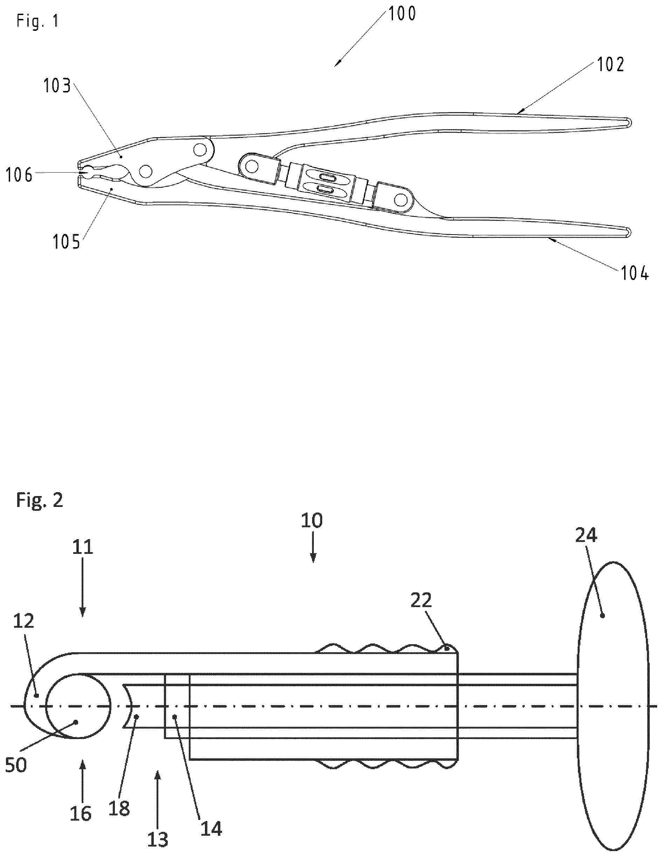

Um den Querstab zu verdrehen, werden Stabfasszangen 100 verwendet, wie sie beispielsweise in Figur 1 gezeigt ist. Eine solche Stabfasszange weist in der Regel zwei Handgriffe 102, 104 auf, die über ein oder mehrere Gelenke miteinander verbunden sind und auf dem Gelenk gegenüberliegenden Seite zwei Greifhebel 103, 105 mit einer Greifaussparung 106, mit der der Stab gehalten wird indem durch Zusammendrücken der Handgriffe eine Kraft auf den Stab wirkt und dieser nicht mehr aus der Greifaussparung der Stabfasszange rutschen kann. Mit Hilfe dieser Zange wird dann der Querstab wie oben beschrieben bewegt.

Ein Problem der herkömmlichen Stabfasszange 100 besteht darin, dass die Greifhebel 103, 105 der Stabfasszange 100 rotatorisch um das Hebelgelenk bewegt werden, wodurch die Greifhebel im Bereich des Querstabs beim Zugreifens und Loslassen des Querstabs raumgreifende Bewegungen durchführen. D.h., für die Bewegungen der Greifhebel muss ausreichend Platz um den Querstab herum bereitgestellt werden und schlimmstenfalls könnte Gewebe zwischen Querstab und Zange eingeklemmt werden. Ferner wird die Kraft an der Greifaussparung zwar über die Hebelwirkung vergrößert, die Handgriffe 102, 104 müssen aber während der gesamten Operation konstant zusammengepresst werden, da sich der Querstab sonst möglicherweise in Richtung seiner Ausgangslage zurückbewegen würde, wenn sich der Griff der Stabfasszange auf den Querstab lockert. Dies ist insbesondere deshalb nachteilig, da der Querstab sich im Patienten befindet, während der in Position gebracht werden soll. US2014107706 A1 offenbart eine Stabfassklemme zum Halten und Bewegen eines Querstabs für Pedikelschrauben umfassend ein erstes stabförmiges Klemmelement und ein zweites stabförmiges Klemmelement, wobei das erste und zweite stabförmige Klemmelement zueinander translatorisch bewegbar sind. DE1810806 A1 offenbart eine Stabfassklemme zum Halten einer Nadel mit einem als Kniehebel ausgebildeten Stangengetriebe.

### Darstellung der Erfindung

Aufgabe der vorliegenden Erfindung ist es daher, eine platzsparende Möglichkeit für eine Stabfassklemme bereitzustellen, die gleichzeitig eine sichere Befestigung am Querstab ermöglicht, und dabei die Gefahr minimiert wird, dass bewegte Elemente mit Gewebe in Kontakt gelangen. Ferner sollte vorzugsweise auch die Haltekraft über die normale Hebelwirkung hinaus erhöht werden können.

Diese Aufgabe wird gelöst von einer Stabfassklemme zum Halten und Bewegen eines Querstabs für Pedikelschrauben, umfassend ein erstes stabförmiges Klemmelement, das an einem distalen Ende eine Aussparung zum Einsetzen des Querstabs aufweist, und ein zweites stabförmiges Klemmelement, das an einem distalen Ende einen Druckabschnitt aufweist und das bewegbar am ersten stabförmigen Klemmelement gelagert ist, wobei das erste und zweite stabförmige Klemmelement zueinander translatorisch bewegbar sind und die Klemmkraft zum Halten des Querstabs mittels der translatorischen Bewegung aufgebracht wird. Stabförmig bedeutet im vorliegenden Fall, dass das Klemmelement mit einer Längserstreckung ausgebildet ist, dabei aber einen beliebigen Querschnitt aufweisen kann, der sich auch über die Längserstreckung ändern kann. Durch die translatorische Bewegung der beiden Klemmelemente und das Fehlen der rotatorischen Bewegung der Klemmelemente kann die Stabfassklemme im distalen Bereich der Klemmelemente mit geringen Dimensionen ausgeführt werden. Und dadurch, dass gerade in dem Abschnitt, der sich an dem Querstab befindet, sehr kleine Dimensionen der Klemmelemente verwirklicht werden können, wird weniger Platz um den zu greifenden Abschnitt des Querstabs benötigt. Darüber hinaus verringert sich auch die Verletzungsgefahr, weil die translatorische Bewegung immer entlang der Stabfassklemme durchgeführt wird und selbst beim Öffnen und Schließen der Klemme im Bereich des Querstabs keine nach außen gerichtete Bewegung erfolgt.

die Klemmelemente der Stabfassklemme sind über ein als Kniehebel ausgebildetes Stangengetriebe miteinander verbunden. Durch den Kniehebel kann die aufgebrachte Kraft über eine normale Hebelwirkung hinaus erhöht, gleichzeitig aber auch die translatorische Bewegung der Klemmelemente zueinander konstruktiv einfach gelöst werden. Der Kniehebel ist dabei eine bevorzugte Lösung, da bei diesem die Klemmkraft stark erhöht werden kann und dabei ein Verrutschen des Stabs beim Halten und Bewegen des Querstabs zuverlässig verhindert werden kann.

Ferner kann die Stabfassklemme eine Selbsthemmung und/oder eine Sperre aufweisen, die ein Lösen der in Halteposition bewegten Klemmelemente verhindert. Dadurch wird die Handhabung während der Operation bzw. des Einstellens und Bewegens des Querstabs stark erleichtert, da die Stabfassklemme nicht dauerhaft mit Kraft beaufschlagt werden muss, um ein Verrutschen des Stabs zu verhindern. Zusätzlich zur Sperre und/oder zusätzlich zur Selbsthemmung kann die Stabfassklemme eine Löseeinrichtung aufweisen, durch die die Selbsthemmung und/oder die Sperre überwindbar ist. Dies ermöglicht oder vereinfacht das Lösen der Selbsthemmung oder der Sperre und verhindert auch, dass beim Lösen der Stabfassklemme vom Querstab oder durch das möglicherweise unkontrollierte Öffnen der Stabfassklemme versehentlich Verletzungen im Operationsbereich erzeugt werden können. Die Löseeinrichtung ist insbesondere bei einer Kniehebelausgestaltung vorteilhaft, da die Kräfte zum Lösen der Selbsthemmung des Kniehebels ebenso groß sein können, wie zum Festsetzen der Stabfassklemme in der Halteposition.

Bevorzugt weist die Aussparung des ersten Klemmelements und/oder der Druckabschnitt des zweiten Klemmelements eine bogenförmige Kontur auf, die insbesondere zur Außenkontur eines Querstabs im Wesentlichen formschlüssig ist. Selbst im Fall einer nicht absolut formschlüssigen Kontur wird die Klemmkraft gleichmäßiger auf den Querstab aufgebracht und sichert so zuverlässig gegen ein Verrutschen des Querstabs ab. Zusätzlich wird auch eine Beschädigung des Querstabs durch zu hohe Krafteinwirkung auf eine sehr geringe Fläche verhindert.

Vorzugsweise ist eines der Klemmelemente innerhalb des anderen Klemmelements gelagert, insbesondere das zweite Klemmelement im ersten Klemmelement. D.h., dass ein Klemmelement zumindest teilweise vom anderen Klemmelement umschlossen ist, vorzugsweise komplett umschlossen. Dadurch wird vermieden, dass Relativbewegungen zwischen den beiden Klemmelementen von außen sicht- oder spürbar sind. Das vermindert dann das Verletzungsrisiko, da die bewegten Teile insbesondere im Bereich des distalen Abschnitts der Stabfassklemme abgedeckt werden.

Die Stabfassklemme umfasst ferner vorzugsweise einen Anschlag, insbesondere einen einstellbaren Anschlag (Schraubenlösung bei Kniehebel), der die Relativbewegung des ersten und zweiten Klemmelements begrenzt. Für eine Kniehebelausgestaltung ist dieser Anschlag insbesondere im Bereich der Handgriffe ausgebildet. Durch die Begrenzung der Relativbewegung kann die Druckkraft auf den Querstab oder auch die Bewegung der Griffhebel gesteuert werden. So kann beispielsweise eine überhöhte Druckkraft oder auch ein Absinken der Druckkraft auf den Querstab verhindert werden.

Der distale Abschnitt der Stabfassklemme des ersten Klemmelements ist auf der der Aussparung gegenüberliegenden Seite eine im Wesentlichen abgerundete, insbesondere bogenförmige, bspw. eine ovale, Außenkontur. Die (Längs-)Kanten am distalen Ende der Stabfassklemme sind ferner vorzugsweise abgerundet. Dadurch wird die Verletzungsgefahr weiter verringert.

Ferner kann bei der Stabfassklemme der Druckabschnitt austauschbar ausgestaltet sein und/oder eine Einsetzaussparung aufweisen. Dadurch können zusätzlich zur Haltefunktion der Stabfassklemme auch andere Funktionen, wie beispielsweise eine Trennfunktion, geschaffen werden.

In einer weiteren Ausgestaltung der Stabfassklemme umfasst die Aussparung des ersten Klemmelements und der Druckabschnitt des zweiten Klemmelements eine Stufe, wodurch auf einfache Weise aus der Stabfassklemme eine Stabtrennklemme wird.

Eine weitere Ausgestaltung der erfinderischen Stabfassklemme betrifft eine Stabfassklemme umfassend ein erstes stabförmiges Klemmelement, das an einem distalen Ende eine Aussparung zum Einsetzen des Querstabs aufweist, und ein zweites stabförmiges Klemmelement, das an einem distalen Ende eine Aussparung zum Einsetzen des Querstabs aufweist und das bewegbar am ersten stabförmigen Klemmelement gelagert ist, wobei die Klemmbewegung der Stabfassklemme durch einen Kniehebelrealisiert wird. Vorteilhafterweise ist in dieser Ausgestaltung auch ein Anschlag vorgesehen, sowie die weiteren vorhergehend beschriebenen Ausgestaltungen.

### Kurze Beschreibung der Figuren

- Figur 1: zeigt eine Stabfasszange aus dem Stand der Technik;
- Figur 2: zeigt eine Schnittansicht einer Ausführungsform der Stabfassklemme gemäß der vorliegenden Erfindung;
- Figur 3: zeigt eine schematische Darstellung einer weiteren Ausführungsform der Stabfassklemme gemäß der vorliegenden Erfindung;
- Figur 4: zeigt eine erfindungsgemäße Stabfassklemme in einem geöffneten Zustand;
- Figur 5: zeigt eine Explosionsansicht der erfindungsgemäßen Stabfassklemme aus Figur 3;
- Figur 6: zeigt eine Detailansicht eines Gelenks mit einer Untermaßeinstellvorrichtung;

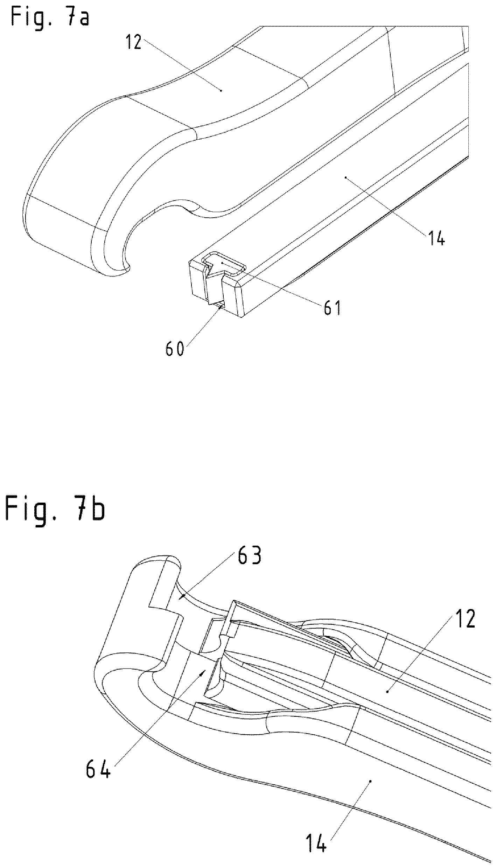

- Figur 7a: zeigt ein distales Ende des zweiten Klemmelements mit einer Einsetzaussparung; und
- Figur 7b: zeigt die distalen Klemmelemente mit einer Stufe.

### Darstellung bevorzugter Ausführungsformen

Im Folgenden wird der Begriff "distal" verwendet, mit dem eine Richtung bezeichnet wird, die vom Anwender weg und zum Patienten hin bezeichnet wird. D.h., das distale Ende der Stabfassklemme ist das Ende, mit dem der Querstab umfasst und gehalten wird. Ferner wird der Begriff "proximal" verwendet, mit dem eine Richtung zum Anwender hin bezeichnet wird.

Eine Stabfassklemme 10 umfasst grundsätzlich ein erstes und ein zweites Klemmelement 12, 14. Die beiden Klemmelemente sind so aneinander gelagert, dass sie eine translatorische Bewegung ausführen können. Am distalen Ende 11 des ersten Klemmelements 12 ist eine Aussparung 16 vorgesehen, in die ein Querstab 50 zum Verbinden von Pedikelschrauben (nicht gezeigt) eingelegt werden kann. Vorzugsweise weist die Aussparung 16 eine abgerundete Form auf, insbesondere eine zumindest abschnittsweise zum Querstab 50 komplementäre/formschlüsige Form. Dadurch wird die Kontaktfläche zwischen Aussparung 16 und Querstab 50 vergrößert. Das distale Ende 13 des zweiten Klemmelements dient als Druckabschnitt 18, mit dem der Querstab 50 in oder an die Aussparung 16 des ersten Klemmelements 12 gedrückt wird und weist vorzugsweise ebenfalls eine abgerundete bzw. zum Querstab 50 zumindest abschnittsweise komplementäre/formschlüssige Form auf. Das erste Klemmelement 12 ist vorzugsweise hakenförmig ausgebildet. Die Hakenform dient zur optimalen Lastverteilung im ersten Klemmelement, wenn die Stabfassklemme mit einer Last beaufschlagt wird. Ferner ist die Außenkontur des ersten Klemmelements 12 vorzugsweise mit abgerundeten Kanten versehen, insbesondere aus einer Seitenansicht oval, wodurch eine längliche, aber dennoch abgerundete Form gewährleistet ist. Die Längskanten des ersten Klemmelements sind ebenfalls vorzugsweise abgerundet. Auch die Kanten des zweiten Klemmelements können abgerundet sein.

Um die Relativbewegung nach außen so wenig wie möglich sichtbar und spürbar zu machen, ist ein Klemmelement 12, 14 vorzugsweise von dem anderen Klemmelement 12, 14 zumindest teilweise umschlossen und in diesem gelagert. Vorzugsweise ist hier das erste Klemmelement 12 mit der Aussparung 16 das Element, das das zweite Klemmelement 14 zumindest teilweise, insbesondere aber auch komplett umschließt.

In den Figuren 1 und 2 bis 5 sind verschiedene Ausführungsformen der vorliegenden Erfindung gezeigt, die alle die oben beschriebenen Klemmelemente aufweisen. Figur 1 zeigt eine Ausführungsform mit einem Gewindestift und die Figuren 2 bis 5 eine Ausführungsform mit einem Stangengetriebe, genauer gesagt einen Kniehebel.

Die in Figur 1 gezeigte Lösung weist die oben genannten ersten und zweiten Klemmelemente 12, 14 auf, wobei das zweite Klemmelement 14 als Gewindestift ausgebildet ist und in eine Bohrung 20 im ersten Klemmelement 12 eingedreht wird. Das distale Ende bzw. der Druckbereich 18 des zweiten Klemmelements 14 ist vorzugsweise rotatorisch relativ bewegbar zum Hauptkörper des zweiten Klemmelements 14 gelagert, so dass sich das distale Ende 18 bzw. der Druckbereich nicht mit dem Hauptkörper des zweiten Klemmelements 14 drehen muss. Am proximalen Ende der Klemmelemente 12, 14 können Handgriffe 22, 24 vorgesehen sein, mit denen das zweite Klemmelement 14 dann in das erste Klemmelement 12 eingeschraubt werden kann und so der in der Aussparung befindliche Querstab 50 befestigt wird.

Der Gewindestab 14 weist vorzugsweise ein Gewinde mit einer Steigung von 0,5 mm bis 5 mm auf. Dieses Gewinde kann als Flachgewinde ausgebildet sein. Die auf den Querstab 50 aufgebrachte Kraft lässt sich durch drehen des Gewindestabs erhöhen, wobei ein Drehgriff 24 mit größerem Durchmesser die Hebelwirkung verbessert, da die Entfernung der Außenabmessung zur Drehachse des Gewindestifts 14 erhöht wird. Die Rotation des Gewindestifts 14 ist nur indirekt für die translatorische Klemmbewegung des Gewindestifts 14 verantwortlich, da die Klemmbewegung selbst keinen rotatorischen Anteil aufweist.

Die Stabfassklemme mit Gewindestift 14 kann einen Anschlag aufweisen, der das Weiterdrehen des Gewindestifts 14 verhindert. Beispielsweise kann dieser durch das Anschlagen des Handgriffs 24 am ersten Klemmkörper 12 ausgebildet sein, kann aber auch über Vorsprünge (nicht gezeigt) in der Aufnahmeaussparung 16 für den Querstab ausgebildet sein, so dass das distale Ende des Gewindestabs 14 in einer vorbestimmten Position gestoppt wird. Dies kann dazu dienen, ein Überdrehen des Gewindestabs 14 zu verhindern, so dass der Querstab 50 nicht durch Aufbringen einer zu großen Kraft beschädigt werden kann. Der Anschlag kann einstellbar ausgebildet sein, bspw. durch eine Schraube. Die Stabfassklemme 10 mit Gewindestift 14 kann eine Sperre aufweisen, die bspw. als Stift ausgestaltet sein kann, der in eine entsprechende Ausnehmung im Handgriff 24 eingesetzt werden kann und die weitere Drehung oder auch das Aufdrehen des Gewindestifts verhindert. Eine selbsthemmende Sperre kann bspw. durch eine sehr flache Gewindesteigung realisiert werden. Da die Klemmkraft nicht nur auf den Querstab, sondern auch auf die Flanken des Gewindes wirkt, ist nach dem ausreichenden Festziehen des Gewindestabs ein selbstständiges Zurückdrehen ohne Unterstützung aufgrund der Haftreibung und der niedrigen Steigung des Gewindes nicht mehr möglich.

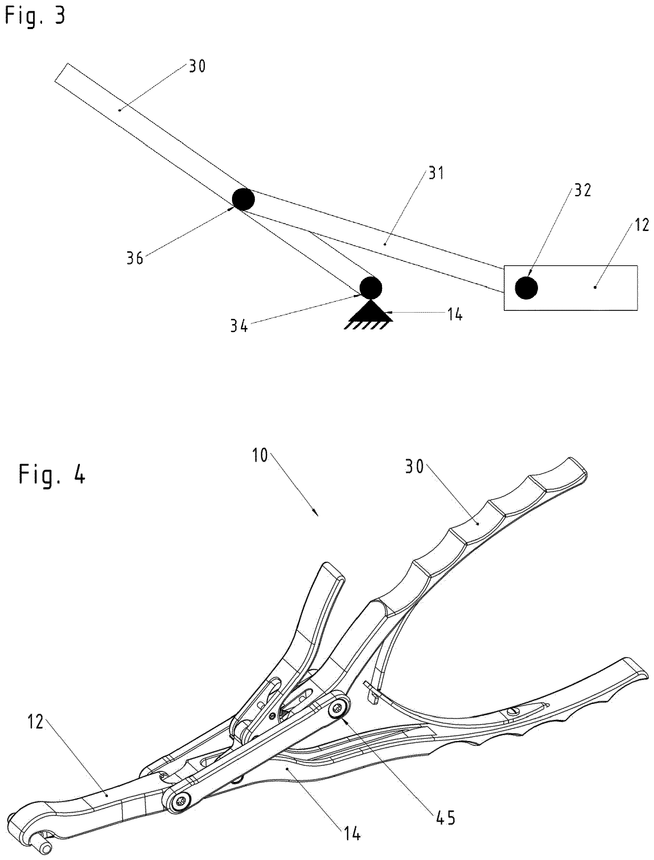

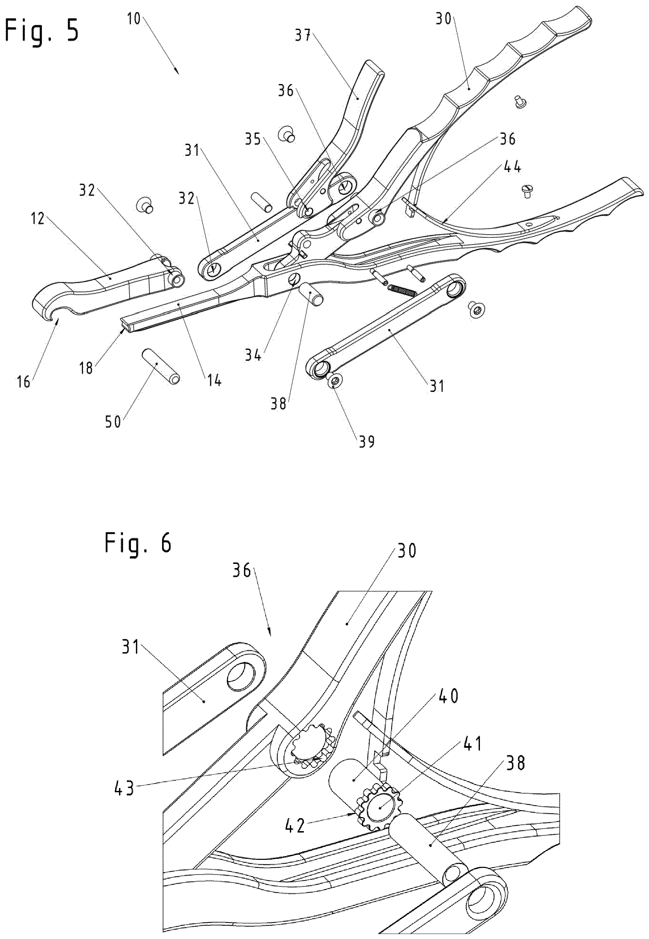

In einer anderen Ausführungsform, die in den Figuren 4a, 4b und 5 gezeigt ist, ist die Stabfassklemme 10 als Stangengetriebe ausgebildet, insbesondere als Kniehebel. Die Funktionsweise des Kniehebels lässt sich anhand von Figur 3 darstellen. Ein erster Hebel (der Griffhebel 30) ist mit einem Gelenk 34 an einem festen Lagerpunkt (hier das zweite Klemmelement 14) drehbar gelagert. Der erste Hebel weist ferner ein weiteres Gelenk 36 auf, an dem ein seitlicher Hebel 31 befestigt ist, der mit einem weiteren Körper (dem ersten Klemmelement 12) über ein weiteres Gelenk 32 verbunden ist. Durch den Kniehebelmechanismus wird eine Verschiebung zwischen dem ersten und zweiten Klemmkörper/Klemmelement 12, 14 realisiert, was in einer hohen Klemmkraft am Stab resultiert. Dabei wird das erste Klemmelement 12 auf Zug beansprucht, während das zweite Klemmelement 14 auf Druck beansprucht wird. Diese Anordnung lässt sich aber auch austauschen, so dass das erste Klemmelement 12 auf Druck und das zweite auf Zug beansprucht wird. Die Klemmkraft steigt beim Kniehebel überproportional, sobald in einer Seitenansicht wie in Figur 3 die drei Gelenke 32, 34, 36 im Wesentlichen in einer Flucht liegen, d.h. in Figur 3 alle drei auf einer waagrechten Linie. Der seitliche Hebel 31 ist dabei für die Kniehebelübersetzung verantwortlich (Hebellänge und Position der Gelenke). Die in den Figuren 3a, 3b und 4 gezeigte Stabfassklemme 10 entspricht der schematischen Darstellung des Kniehebels aus Figur 3, wobei der feste Lagerpunkt das zweite Klemmelement 14 und der Klemmkörper das erste Klemmelement 12 darstellen.

Der erste Klemmkörper weist das Gelenk 32 auf, das mit dem seitlichen Hebel 31 verbunden ist. Der seitliche Hebel 31 ist vorliegend auf beiden Seiten der Stabfassklemme 10 ausgebildet um die aufgebrachten Kräfte gleichmäßiger übertragen zu können. Die Klemmelemente 12, 14 und der seitliche Hebel 31 sind miteinander an den Gelenken über Gewindebolzen 38 und entsprechende Schrauben/Muttern 39 verbunden. So kann gewährleistet werden, dass sich durch die Bewegung der Hebel 30, 31 und das Verdrehen an ihren Gelenkpunkten 32, 34, 36 die Verbindung nicht zufällig löst, da die Schrauben 39 und die Gewindebolzen 38 frei drehbar an den Gelenkpunkten 32, 34, 36 in den Klemmelementen 12, 14 und den Hebeln 30, 31 gelagert sind. Der seitliche Hebel 31 kann an einem oder beiden Gelenken 32, 36, mit denen er mit dem Griffhebel 30 bzw. dem ersten Klemmelement 12 verbunden ist, eine Einstellvorrichtung 40 besitzen, mit der die Klemmkraft eingestellt werden kann. In Figur 6 ist in einer Vergrößerung eine solche Vorrichtung als Einstellbuchse 40 gezeigt. Die Buchse 40 besitzt eine innere Bohrung 41 für den Bolzen 38 und/oder die Schrauben 39, mit denen der seitliche Hebel 31 am Griffhebel 30 der Stabfassklemme 10 verbunden wird. Am Außenumfang ist eine Vielzahl von Zähnen 42 angeordnet, die mit entsprechenden Aussparungen 43 an der Durchgangsbohrung des Gelenks 36 des Griffhebels 30 in Eingriff gebracht werden können. Die Bohrung 41 für den Bolzen 38 ist in der Einstellbuchse 40 exzentrisch angeordnet. Dadurch kann die Einstellbuchse 40 aus der Aussparung 43 des Hebels 30 herausgenommen, verdreht und wieder eingesetzt werden, wobei sich durch die exzentrische Anordnung der Bohrung 41 in der Buchse 40 der Verbindungspunkt des seitlichen Hebels 31 in Bezug auf den Griffhebel 30 verschiebt. Beispielsweise kann die Einstellbuchse 40 mit der Exzentrizität nach hinten eingebaut werden und so ein Untermaß erreicht werden, das größer ist, als wenn die Einstellbuchse 40 mit der Exzentrizität nach vorne eingebaut würde. Je nach Einbauposition der Einstellbuchse kann somit das Untermaß zwischen dem ersten und dem zweiten Klemmelement 12, 14 variiert werden. So kann gewährleistet werden, dass die Radien der Klemmelemente 12, 14 den gewünschten Abstand von beispielsweise einem Querstabdurchmesser von 4,5 mm in der Totpunktlage aufweisen. Vorzugsweise weist die Einstellbuchse 12 Zähne und die Durchgangsbohrung des Gelenks 36 zwölf Aussparungen auf. Diese Anzahl lässt eine hinreichen genaue Einstellmöglichkeit zu, ist aber konstruktiv noch einfach herzustellen. Eine solche Einstellbuchse kann an jedem der Gelenke 32, 34, 36 vorgesehen sein.

Es ist vorteilhaft auch die erfindungsgemäße Stabfassklemme mit zwei Griffhebeln auszubilden, die mit einer Hand zueinander gedrückt werden können und so die Klemmkraft zu erzeugen, die auf den Querstab 50 aufgebracht wird. Im vorliegenden Fall werden als Handgriffe zum einen der Griffhebel 30 verwendet, zum anderen der proximale Abschnitt des zweiten Klemmelements.

Die Stabfassklemme kann auch in dieser Ausführungsform einen Anschlag 45 aufweisen, der zur Aufrechterhaltung des Kraftzenits dient. In der vorliegenden Ausführungsform ist der Anschlag 45 an dem Gelenk 36 zwischen Griffhebel 30 und seitlichem Hebel 31 und der Verlängerung des zweiten Klemmelements 14 ausgebildet, so dass der das Gelenk 36 des Griffhebels 30 an den zweiten Griffhebel des zweiten Klemmelements 14 anschlägt. Dadurch wird verhindert, dass der Griffhebel 30 beim Herunterdrücken über den Totpunkt des Kniehebels hinaus gedrückt werden kann und die auf den Querstab 50 wirkende Klemmkraft wieder verringert würde, so dass die Maximalkraft auf den Querstab gehalten werden kann. Ein Anschlag kann aber auch einstellbar ausgestaltet werden. Dazu kann beispielsweise in der in Figur 4 gezeigten Ausführungsform in dem zweiten Griffhebel eine Schraube vorgesehen sein, die je nachdem wie tief sie eingeschraubt wird über den zweiten Griffhebel in Richtung Gelenk 36 hervorstehen kann.

Die Maximalkraft der Stabfassklemme kann über eine geometrischen Änderung des Kniehebels eingestellt werden, welche einerseits durch die Längenadaption der seitlichen Hebel (31) und anderseits durch den Versatz der Gelenkpositionen (32, 34, 36) erzielt werden kann. In der abgebildeten Variante wird die oben genannte Verstelleinrichtung 40 verwendet.

Durch die Verstelleinrichtung kann vorliegend die Ausrichtung der Gelenke leicht versetzt werden um in die richtige Position für die Selbsthemmung des Kniehebels zu gelangen. Insbesondere zusammen mit der Verstelleinrichtung 40 ist der Verstellbare Anschlag hilfreich. Denn dadurch, dass der Anschlag verstellbar ist, kann die Positionsverschiebung ausgeglichen werden und beim Aufbringen der Kraft auf den Querstab das erste und zweite Klemmelement 12, 14 in der richtigen Position gehalten werden.

Eine Sperre kann beispielsweise durch eine (Metall-)Schlaufe ausgebildet sein, die an einem Handgriff befestigt ist und um den anderen Handgriff herum gelegt wird, so dass ein selbstständiges Öffnen nicht möglich ist. Eine Sperre kann zusätzlich zu einer Selbsthemmung gegeben sein, wie sie bei der Kniehebelkonstruktion schon inhärent gegeben ist, wenn die Gelenke in einer Flucht liegen. Um eine solche Selbsthemmung zu lösen, muss eine relativ große Kraft aufgebracht werden, nämlich die nahezu gleiche Kraft wie zum Festsetzen der Stabfassklemme in die geschlossene Position. Vorzugsweise ist daher für die einfachere Handhabung eine Lösevorrichtung 37 vorgesehen. Diese ist beispielsweise als Lösehebel 37 ausgebildet, und in den Figuren 3a, 3b und 4 gezeigt. Dieser Lösehebel 37 ist am Griffhebel 30 in einem Bereich befestigt, an dem der Griffhebel 30 und das zweite Klemmelement 14 in der geschlossenen Position aneinander anliegen. In der vorliegenden Ausführungsform weist der Lösehebel 37 auf der proximalen Seite der Gelenkverbindung 35 mit dem Griffhebel 30 einen Vorsprung 33 auf, der nach unten hervor steht und in der geschlossenen Position am zweiten Klemmelement 14 anliegt. Wenn nun die selbsthemmende Sperre des Kniehebels gelöst werden soll, wird der Lösehebel 37 nach unten gedrückt und über die Hebelwirkung des Vorsprungs 33 des Lösehebels 37 das zweite Klemmelement 14 nach unten weg von dem Griffhebel 30 gedrückt. Dadurch werden die Gelenke 32, 34, 36 des Kniehebels aus der Flucht gebracht und die Stabfassklemme 10 kann außerhalb des Kraftzenits geöffnet werden. Der Lösehebel 37 kann aber auch anders ausgebildet sein, beispielsweise kann er auf der distalen Seite des Verbindungsgelenks 35 einen Vorsprung 33 aufweisen, so dass der Lösehebel 37 nach oben gehoben werden muss um das zweite Klemmelement 14 vom Griffhebel 30 weg zu drücken. Die Lösung mit dem Vorsprung auf der proximalen Seite ist jedoch vorteilhaft, da der Lösehebel 37 einfacher nach unten gedrückt werden kann, wenn man die Stabfassklemme 10 an den Griffabschnitten einhändig hält.

Zwischen den beiden Griffhebeln, d.h., dem Griffhebel 30 und dem Griffhebel des zweiten Klemmelements 14, kann eine Feder 44 vorgesehen sein, die die Griffe auseinanderdrückt und die Stabfassklemme 10 so in der offenen Position hält. Die Feder ist in dem in den Figuren gezeigten Ausführungsbeispiel durch zwei Blattfedern ausgebildet, die an ihrem Ende jeweils eine Aussparung und einen komplementären Einsatz aufweisen, die hier einfach ineinander gehakt werden. Ebenfalls ist an einer Feder eine eine rechteckförmige Aussparung an einem Ende und ein T-förmiger Vorsprung am Ende der anderen Feder vorstellbar, die in die rechteckförmige Aussparung eingesetzt werden kann.

Der Kniehebel kann auf verschiedene Arten ausgebildet werden, so kann beispielsweise das zweite Klemmelement 14 die Aufnahmeaussparung 16 für den Querstab 50 aufweisen und das erste Klemmelement 12 den Druckbereich 18. Ebenso kann das erste Klemmelement 12 mit einem Griffhebel ausgebildet sein.

Im Gebrauch wird der Querstab 50 in die Aufnahmeaussparung 16 des ersten Klemmelements 12 eingehakt und dann der Griffbereich des zweiten Klemmelements 14 und der Griffhebel 30 zusammengedrückt. Die dafür notwendige Kraft erhöht sich stetig, je näher die Gelenke 32, 34, 36 in eine Flucht kommen. Erst wenn die Gelenke an ihren Totpunkt gelangen, schließt sich die Stabfassklemme von selbst und die Selbsthemmung verhindert ein versehentliches Öffnen.

Das zweite Klemmelement der Stabfassklemme kann eine Aussparung 60 aufweisen, in die beispielsweise ein Dorn 61 oder ein anderes Einsatzelement eingesetzt werden kann. Der Dorneinsatz 61 weist eine hervorstehende Spitze auf, mit der ein Querstab 50 abgetrennt werden kann. Dies ist gerade durch die äußerst hohe Kraft möglich, die mit der vorliegenden Stabfassklemme aufgebracht werden kann. In einer anderen Ausgestaltung weist sowohl die Aussparung 16 des ersten Klemmelements 12 als auch der Druckabschnitt 18 des zweiten Klemmelements 14 eine Stufe 63, 64 auf, wobei die Stufen einander entsprechen. D.h. dass der Abstand zwischen den zueinander gerichteten Flächen der Stufen des ersten und zweiten Klemmelements im Wesentlichen gleich bleibt. Diese Stufe dient ebenfalls zum Abtrennen eines Querstabs.

## Patentansprüche

1. Stabfassklemme (10) zum Halten und Bewegen eines Querstabs (50) für Pedikelschrauben, umfassend
ein erstes stabförmiges Klemmelement (12), das an einem distalen Ende (11) eine Aussparung (16) zum Einsetzen des Querstabs (50) aufweist; und
ein zweites stabförmiges Klemmelement (14), das an einem distalen Ende (13) einen Druckabschnitt (18) aufweist und das bewegbar am ersten stabförmigen Klemmelement (12) gelagert ist;
das erste und zweite stabförmige Klemmelement (12, 14) zueinander translatorisch bewegbar sind und die Klemmkraft zum Halten des Querstabs (50) mittels der translatorischen Bewegung aufgebracht wird
**dadurch gekennzeichnet, dass**
die Klemmelemente (12, 14) über ein als Kniehebel ausgebildetes Stangengetriebe miteinander verbunden sind.

2. Stabfassklemme (10) nach Anspruch 1, die ferner eine Selbsthemmung und/oder eine Sperre aufweist, die ein Lösen der in Halteposition bewegten Klemmelemente (12, 14) verhindert.

3. Stabfassklemme (10) nach Anspruch 1 oder 2, ferner umfassend eine Löseeinrichtung (37), durch die die Selbsthemmung und/oder die Sperre lösbar ist.

4. Stabfassklemme (10) nach einem der vorhergehenden Ansprüche, bei der die Aussparung (16) des ersten Klemmelements (12) und/oder der Druckabschnitt (18) des zweiten Klemmelements (14) eine bogenförmige Kontur aufweist.

5. Stabfassklemme (10) nach einem der vorhergehenden Ansprüche, bei der ein Klemmelement (12, 14) innerhalb des anderen Klemmelements (12, 14) gelagert ist.

6. Stabfassklemme (10) nach einem der vorhergehenden Ansprüche, ferner umfassend einen Anschlag (45), der die Relativbewegung des ersten und zweiten Klemmelements (12, 14) begrenzt.

7. Stabfassklemme (10) nach einem der vorhergehenden Ansprüche, bei der der Druckabschnitt (18) austauschbar ausgestaltet ist oder eine Einsetzaussparung aufweist.

8. Stabfassklemme (10) nach einem der vorhergehenden Ansprüche, bei der das distale Ende (11) des ersten Klemmelements (12) eine im Wesentlichen ovale Außenkontur aufweist.

9. Stabfassklemme (10) nach einem der vorhergehenden Ansprüche, ferner umfassend eine Einstellvorrichtung (40) zum Einstellen der Hebellänge und/oder der Position der Gelenke (32, 34, 36).

10. Stabfassklemme (10) nach einem der vorhergehenden Ansprüche, bei der der Druckabschnitt (18) des zweiten Klemmelements (14) eine Einsetzaussparung (60) aufweist oder die Aussparung (16) des ersten Klemmelements (12) und der Druckabschnitt (18) des zweiten Klemmelements (14) eine Stufe aufweisen.

## Claims

1. Rod-type barrel clamp (10) for holding and moving a cross rod (50) for pedicle screws comprising
a first rod-shaped clamping member (12) having a recess (16) at a distal end (11) for insertion of the cross rod (50); and
a second rod-shaped clamping element (14) having a pressure section (18) at a distal end (13) and being movably mounted on the first rod-shaped clamping element (12);
the first and second rod-shaped clamping element (12, 14) are translationally movable relative to each other and the clamping force for holding the cross rod (50) is applied by means of the translational movement
**characterized in that**
the clamping elements (12, 14) are connected to one another via a rod gear, which is configured as a toggle lever.

2. Rod-type barrel clamp (10) according to claim 1, which further comprises a self-locking and/or a barrier, which prevents a release of the clamping elements (12, 14) moved in holding position.

3. Rod-type barrel clamp (10) according to claim 1 or 2, further comprising a release device (37) by which the self-locking and/or the barrier is releasable.

4. Rod-type barrel clamp (10) according to any one of the preceding claims, wherein the recess (16) of the first clamping element (12) and/or the pressure section (18) of the second clamping element (14) has an arcuate contour.

5. Rod-type barrel clamp (10) according to any one of the preceding claims, wherein a clamping element (12, 14) is mounted inside the other clamping element (12, 14).

6. Rod-type barrel clamp (10) according to any one of the preceding claims, further comprising a stopper (45), which limits the relative movement of the first and second clamping element (12, 14).

7. Rod-type barrel clamp (10) according to any one of the preceding claims, wherein the pressure section (18) is interchangeably configured or has a recess for insertion.

8. Rod-type barrel clamp (10) according to any one of the preceding claims, wherein the distal end (11) of the first clamping element (12) has a substantially oval outer contour.

9. Rod-type barrel clamp (10) according to any one of the preceding claims, further comprising an adjusting device (40) for adjusting the lever length and/or the position of the joints (32, 34, 36).

10. Rod-type barrel clamp (10) according to any one of the preceding claims, wherein the pressure section (18) of the second clamping element (14) comprises an insertion recess (60) or the recess (16) of the first clamping element (12) and the pressure section (18) of the second clamping element (14) comprise a step.

## Revendications

1. Collier de serrage à tige (10) pour le maintien et le déplacement d'une tige transversale (50) pour vis pédiculaires, comprenant
un premier élément de serrage en forme de tige (12) ayant un évidement (16) à une extrémité distale (11) pour insérer la tige transversale (50) ; et
un deuxième élément de serrage en forme de tige (14) ayant une section de pression (18) à une extrémité distale (13) et étant monté de manière mobile sur le premier élément de serrage en forme de tige (12) ;
les premier et deuxième éléments de serrage (12, 14) en forme de tige sont mobiles en translation l'un par rapport à l'autre et la force de serrage pour maintenir la tige transversale (50) est appliquée au moyen du mouvement de translation
**caractérisé en ce que**
les éléments de serrage (12, 14) sont reliés l'un à l'autre par l'intermédiaire d'un engrenage à tige, qui est configuré comme levier à bascule.

2. Collier de serrage (10) en forme de tige selon la revendication 1, qui comprend en outre un verrouillage automatique et/ou une barrière, qui empêche un desserrage des éléments de serrage (12, 14) déplacés en position de maintien.

3. Collier de serrage à tige (10) selon la revendication 1 ou 2, comprenant en outre un dispositif de déverrouillage (37) par lequel le verrouillage automatique et/ou la barrière peut être déverrouillé.

4. Collier de serrage (10) en forme de tige selon l'une quelconque des revendications précédentes, dans lequel l'évidement (16) du premier élément de serrage (12) et/ou la section de pression (18) du second élément de serrage (14) présente un contour arqué.

5. Collier de serrage (10) en forme de tige selon l'une quelconque des revendications précédentes, dans lequel un élément de serrage (12, 14) est monté à l'intérieur de l'autre élément de serrage (12, 14).

6. Collier de serrage (10) en forme de tige selon l'une quelconque des revendications précédentes, comprenant en outre un bouchon (45), qui limite le mouvement relatif du premier et du second élément de serrage (12, 14).

7. Collier de serrage (10) en forme de tige selon l'une quelconque des revendications précédentes, dans lequel la section de pression (18) est configurée de manière interchangeable ou présente un évidement pour insertion.

8. Collier de serrage (10) en forme de tige selon l'une quelconque des revendications précédentes, dans lequel l'extrémité distale (11) du premier élément de serrage (12) présente un contour extérieur sensiblement ovale.

9. Collier de serrage à tige (10) selon l'une quelconque des revendications précédentes, comprenant en outre un dispositif de réglage (40) pour régler la longueur du levier et/ou la position des articulations (32, 34, 36).

10. Collier de serrage (10) en forme de tige selon l'une quelconque des revendications précédentes, dans lequel la section de pression (18) du deuxième élément de serrage (14) comprend un évidement d'insertion (60) ou l'évidement (16) du premier élément de serrage (12) et la section de pression (18) du deuxième élément de serrage (14) comprennent une marche.
